Europäisches Patentamt

European Patent Office

Office européen des brevets

(19)

(11) EP 0 844 483 A1

(12) **DEMANDE DE BREVET EUROPEEN**

(43) Date de publication:
27.05.1998 Bulletin 1998/22

(51) Int Cl.⁶: **G01N 33/50**, C12Q 1/02

(21) Numéro de dépôt: 97420214.5

(22) Date de dépôt: 18.11.1997

(84) Etats contractants désignés:
AT BE CH DE DK ES FI FR GB GR IE IT LI LU MC NL PT SE
Etats d'extension désignés:
AL LT LV MK RO SI

(30) Priorité: 20.11.1996 FR 9614424

(71) Demandeur: PASTEUR MERIEUX SERUMS ET VACCINS
69007 Lyon (FR)

(72) Inventeurs:
• **Kourilsky, Philippe**
75001 Paris (FR)
• **Bachy, Monique**
69440 Ste Catherine (FR)
• **Trannoy, Emanuelle**
69005 Lyon (FR)

(74) Mandataire: **Kerneis, Danièle et al**
**Pasteur Mérieux Connaught,**
**Direction de la Propriété Intellectuelle,**
**BP 7046**
**69348 Lyon Cedex (FR)**

(54) **Méthode de mise en évidence de l'activité cytotoxique de cellules**

(57) L'invention est relative à une méthode de mise en évidence de l'activité cytotoxique de cellules effectrices, selon laquelle on met en contact les cellules effectrices et des cellules-cibles et on apprécie la cytotoxicité par dosage d'un élément marqueur présent dans le cytoplasme des cellules-cibles et libéré dans le milieu extracellulaire en cas d'activité cytotoxique des cellules effectrices vis-à-vis des cellules-cibles , caractérisée en ce qu'on introduit au préalable dans les cellules-cibles au moins un gêne codant pour ledit élément marqueur.

EP 0 844 483 A1

**Description**

L'invention concerne le domaine des techniques de dosage. Plus particulièrement, l'invention concerne une méthode pour la mise en évidence de l'activité cytotoxique de cellules.

L'étude de l'activité cytotoxique des cellules, qu'elle soit spécifique, (telle que celle due aux lymphocytes T $CD_{4+}$, $CD_{8+}$) ou non spécifique (telle que celle due aux cellules NK, aux macrophages...) impose de pouvoir mettre en évidence cette activité, ce qui présente certaines difficultés.

Il existe, dans l'art antérieur, des techniques connues pour mettre en évidence cette activité qui sont basées sur l'appréciation de la lyse de cellules-cibles par les cellules dont on veut mettre en évidence le pouvoir cytotoxique et que l'on qualifie de cellules effectrices. Lorsqu'elles sont lysées, les cellules cibles libèrent dans le milieu extracellulaire une partie de leurs constituants. Le dosage d'au moins un de ces constituants permet alors d'apprécier l'importance de la lyse intervenue, et par conséquent, le pouvoir cytotoxique des cellules effectrices.

Selon une méthode décrite par Brunner K.T., Manuel J., Cerottini J.C. et Chapuis B. en 1968 dans Immunology, _14_, _181_, on utilise comme cellules-cibles des cellules auxquelles on a préalablement fait ingérer du chrome radioactif $^{51}Cr$ ; ce chrome est libéré lors de la lyse cellulaire et peut alors être dosé. Ce test présente l'avantage d'être sensible ; il a, cependant, pour inconvénient majeur de conduire à la manipulation d'éléments radioactifs. En outre, certaines des cellules-cibles utilisées, telles que les blastes PHA (lymphocytes du sang périphérique activés par de la PHA) incorporent faiblement ce $^{51}Cr$ et d'autres cellules le libèrent par contre souvent de façon spontanée, ce qui rend difficile l'interprétation des résultats.

Afin de s'affranchir des problèmes liés à la radioactivité, d'autres méthodes de l'art antérieur ont utilisé la détection d'enzymes initialement présentes dans les cellules vivantes et libérées dans le milieu extracellulaire après la mort de la cellule, telle que la LDH ou lactate déshydrogénase (cf. notamment Korzeniewski C. and Callewaert D. (1983), Journal of Immunological methods, _64_, _313_). Ce type de méthode présente cependant l'inconvénient de manquer de spécificité. En effet, les enzymes dosées sont présentes dans toutes les cellules et leur libération dans le milieu extracellulaire peut être provoquée, non seulement par la lyse des cellules-cibles mais aussi, tout simplement par la mort spontanée d'autres cellules. La mesure de l'activité enzymatique du milieu extracellulaire ne reflète donc pas uniquement l'activité cytotoxique des cellules effectrices.

Selon l'art antérieur, il a également été proposé des techniques basées sur l'introduction à l'intérieur de cellules-cibles vivantes d'un marqueur coloré ou fluorescent qui sera libéré lors de la lyse cellulaire. Cependant, il est possible que certains composés du milieu de culture interfèrent avec les tests de révélation de ces marqueurs, ou encore que ces marqueurs présentent une certaine toxicité vis-à-vis des cellules.

Il existe donc un besoin d'une méthode fiable, ne faisant pas appel à la manipulation d'éléments radioactifs, pour mettre en évidence l'activité cytotoxique de cellules.

Pour répondre à ce besoin, la présente invention a pour objet une méthode de mise en évidence de l'activité cytotoxique de cellules effectrices, selon laquelle on met en contact les cellules effectrices et des cellules-cibles et on apprécie la cytotoxicité par dosage d'un élément marqueur présent dans le cytoplasme des cellules-cibles et libéré dans le milieu extracellulaire en cas d'activité cytotoxique des cellules effectrices vis-à-vis des cellules-cibles, caractérisée en ce qu'on introduit au préalable dans les cellules-cibles au moins un gène codant pour ledit élément marqueur.

Selon un mode particulièrement avantageux de réalisation de la méthode de l'invention, on introduit en outre dans les cellules-cibles au moins un gène codant pour un antigène particulier vis-à-vis duquel on souhaite évaluer l'activité cytotoxique des cellules effectrices.

Ainsi, la méthode selon l'invention permet d'évaluer non seulement l'activité cytotoxique non spécifique de cellules effectrices mais également l'activité cytotoxique spécifique d'un antigène particulier. Cette possibilité est d'un intérêt particulier lorsque l'on veut, par exemple, connaître la capacité qu'a un antigène présent dans une composition vaccinale de provoquer une réaction du système immunitaire conduisant à une activation et une prolifération de lymphocytes T cytotoxiques spécifiques de cet antigène.

Selon une caractéristique particulière de l'invention, le gène codant pour l'élément marqueur et le gène codant pour l'antigène particulier sont introduits dans les cellules-cibles au moyen d'un vecteur unique. Ainsi, toute cellule lysée spécifiquement sera mise en évidence par la libération simultanée de l'élément marqueur.

Selon une autre caractéristique de l'invention, l'élément marqueur est une enzyme qui peut, notamment, être la β-galactosidase. Ainsi, il est relativement facile, grâce à des méthodes communément utilisées, de doser la quantité d'enzyme libérée et donc d'apprécier le pouvoir cytotoxique des cellules effectrices.

D'autres avantages et particularités de la présente invention apparaîtront à la lecture de la description qui va suivre, en référence aux figures qui illustrent des résultats obtenus avec la méthode selon l'invention et avec une méthode selon l'art antérieur.

Les figures 1a et 1b illustrent des résultats obtenus avec des cellules-cibles sensibilisées par incubation soit avec le peptide NP, soit avec le peptide HA, et marquées par un seul élément (figure 1a) ou par 2 éléments (figure 1b).

La figure 2 illustre des résultats obtenus avec des cellules-cibles doublement marquées, co-infectées par 2 vecteurs différents un pour la βgalactosidase et un pour l'antigène particulier.

La figure 3 illustre des résultats obtenus avec des cellules-cibles doublement marquées, infectées par un vecteur digénique permettant l'expression à la fois de la βgalactosidase et de l'antigène particulier.

Les figures 4 et 5 illustrent des résultats obtenus avec des cellules-cibles humaines, co-infectées par 2 vecteurs différents un pour la βgalactosidase et un pour l'antigène particulier.

Selon l'invention, il est possible d'évaluer le pouvoir cytotoxique de cellules que l'on appelle cellules effectrices vis-à-vis de cellules que l'on appelle cellules-cibles. les cellules effectrices peuvent être des macrophages, des cellules NK (pour Natural Killer), des cellules LAK (pour "Lymphokine Activated Killer"), des cellules cytotoxiques dépendantes d'anticorps ou cellules K (pour Killer), des cellules T cytotoxiques, ou toute autre cellule à activité cytotoxique, qu'elle soit spécifique ou non. Ces cellules effectrices peuvent provenir du sang périphérique, ce qui est généralement le cas lorsqu'il s'agit de cellules humaines, ou de tout autre organe ; il peut notamment s'agir de cellules spléniques de souris.

Afin de permettre une meilleure détection de l'activité cytotoxique, il est parfois nécessaire de procéder à une amplification in-vitro des cellules cytotoxiques présentes dans les échantillons à analyser. Cette stimulation peut être réalisée par toute méthode connue ; on peut notamment, dans le cas où on essaie de mettre en évidence une activité cytotoxique spécifique d'un antigène du virus de la grippe, réaliser une stimulation des cellules effectrices, par mise en culture in vitro des cellules effectrices avec des cellules stimulantes qui sont des cellules présentatrices d'antigènes (CPA) contenues dans le sang ou tout autre organe et qui ont été au préalable infectées avec du virus vivant de la grippe tel que le virus NIB 16. Après un temps de culture adéquate, par exemple une semaine, il est possible de procéder à une seconde stimulation réalisée de la même façon que la première et/ou par addition de cytokines et conduisant également à une mise en culture des cellules effectrices avec les cellules stimulantes. Lors de ces étapes de stimulation, ce sont essentiellement les cellules effectrices capables de reconnaître l'antigène spécifique à la surface des CPA qui sont stimulées et amplifiées et qui sont donc maintenues en vie.

Les cellules effectrices utilisées pour mettre en oeuvre la méthode selon l'invention peuvent être les cellules obtenues ex-vivo après la mise en culture effectuée lors de la première stimulation ou bien les cellules obtenues après la mise en culture effectuée lors de la seconde stimulation.

Un exemple de cellules effectrices dont on veut mettre en évidence l'activité cytotoxique est constitué par un échantillon de lymphocytes T du sang d'un être humain à qui on a administré au préalable un antigène vaccinal. En effet, lors de la mise au point de vaccin, il est nécessaire de vérifier la réaction du système immunitaire au vaccin administré ; cette réaction peut être de type humorale (on dose alors la quantité d'anticorps sécrétés en réponse au vaccin administré) ou à médiation cellulaire ; dans ce dernier cas, il convient d'évaluer si l'administration du vaccin a induit des cellules cytotoxiques, et si oui, si la cytotoxicité induite est une cytotoxicité spécifique de la (ou des) valence (s) vaccinale(s) administrée(s), ou s'il s'agit d'une cytotoxicité non spécifique.

La nature des cellules-cibles convenant aux fins de l'invention varie en fonction de la nature des cellules effectrices dont on veut apprécier le pouvoir cytotoxique.

S'il s'agit de cellules effectrices à activité cytotoxique non spécifique, on peut utiliser comme cellules-cibles des lignées cellulaires telles que :

- les YAC-1 qui sont des lymphomes de souris, si les cellules effectrices sont d'origine murine.
- les K562 qui sont des cellules d'érythroleucémie humaine, si les cellules effectrices sont d'origine humaine,

Si, par contre, il s'agit de cellules effectrices à activité cytotoxique dépendant du Complexe Majeur d'Histocompatibilité (CMH), il sera possible d'utiliser les cellules suivantes :

a) dans le cas où les cellules effectrices sont d'origine murine :

- les P815 qui sont des cellules provenant d'un mastocytome de souris,
- les TA3 qui sont des hybridomes B de souris.

b) dans le cas où les cellules effectrices sont d'origine humaine :

- des fibroblastes issus par exemple de peau humaine,
- des PBL ou lymphocytes issus de sang périphérique humain,
- des blastes PHA qui sont des PBL activés par de la Phytohémagglutinine,
- des lignées EBV qui sont des PBL transformés par du virus Epstein Barr, mais qui présentent l'inconvénient de nécessiter un délai d'environ 6 semaines pour les établir.

Toute autre cellule capable d'être lysée par cytotoxicité est également susceptible de convenir. De préférence, on utilisera des cellules-cibles aptes à présenter un antigène ou du moins des peptides épitopes d'un antigène déterminé en association avec les antigènes du CMH afin de pouvoir apprécier le pouvoir cytotoxique des cellules-cibles vis-à-vis de cet antigène.

Selon l'invention, on incorpore aux cellules-cibles le gène d'un élément qui pourra être exprimé par la cellule, et qui sera libéré dans le milieu extracellulaire lors de la lyse cellulaire. Cet élément marqueur est constitué par toute protéine susceptible d'être exprimée par les cellules-cibles et qu'il est possible de doser lors de sa libération dans le milieu extracellulaire. De préférence, la protéine utilisée est une protéine cytoplasmique non toxique pour les cellules, et non présente à l'état naturel dans les cellules du test. Parmi les protéines qui conviennent, on peut citer la GFP (ou Green Fluorescent Protein) ou les enzymes, notamment les enzymes thermostables telles que la β-galactosidase, la luciférase ou une phosphatase alcaline. La β-galactosidase convient particulièrement bien car il est possible, grâce à son activité sur différents substrats, de la doser par différentes techniques : colorimétrie, fluorimétrie ou luminescence.

L'incorporation aux cellules cibles du gène codant pour cet élément marqueur peut être effectuée par tout moyen approprié et par exemple par transfection d'un plasmide ou par infection d'un vecteur viral recombinant contenant le gène. Les vecteurs qui conviennent à cette fin doivent pouvoir être produits facilement, être stables, être capables de recevoir plusieurs kbs (kilobase) de matériel génétique exogène ; ils doivent également être capables d'infecter un large échantillon de cellules.

On a remarqué qu'aux fins de l'invention, convenaient bien des vecteurs d'expression obtenus à partir de virus Pox, par exemple du virus de la vaccine éventuellement modifié (vecteur NYVAC de Virogenetics, cf Virology 188 p. 217 - 232 (1992) Tartaglia et al "NYVAC : a Highly Attenuated Strain of Vaccinia Virus") ou du virus canarypox (vecteur ALVAC de Virogenetics). Ces vecteurs peuvent être obtenus par recombinaison homologue ainsi que cela a été décrit dans Enzymology Vol 15 p.545 - 563 (1987) par Antonia PICCINI, Marion E. PERKUS et Enzo PAOLETTI dans "Vaccinia Virus as an Expression Vector". Ils peuvent être modifiés pour augmenter la sécurité de leur emploi selon toute technique connue et notamment selon les techniques décrites dans le brevet US 5,494,807.

Les conditions d'introduction dans les cellules-cibles du gène codant pour l'élément marqueur (et notamment les valeurs de multiplicité d'infection) sont adaptées en fonction de la nature des cellules-cibles et du vecteur utilisé afin d'obtenir une méthode aussi sensible que possible. Dans le cas où l'élément marqueur est la β-galactosidase dont l'activité est mesurée par "Glow Luminescence" avec le substrat AMPGD, on peut, par exemple, fixer le seuil de sensibilité comme étant le nombre minimal de cellules donnant, lorsqu'on lit la luminescence, un écart d'au moins un log entre le résultat obtenu pour les cellules infectées et le résultat obtenu pour les cellules témoins non infectées. Il est naturellement avantageux d'utiliser des conditions telles que le nombre de cellules-cibles soit inférieur ou égal à celui nécessaire pour la mise en oeuvre de la méthode au chrome radioactif.

Selon un mode particulier, l'activité cytotoxique que l'on veut mettre en évidence est une activité cytotoxique spécifique d'un antigène particulier. Dans ce cas, les cellules-cibles peuvent être sensibilisées par incubation avec l'antigène particulier, ou du moins avec des peptides épitopes de cet antigène. Selon l'invention, on préfère introduire dans les cellules-cibles le gène codant pour l'antigène déterminé dont les épitopes seront exprimés à la surface de la cellule en association avec les antigènes du CMH. Ce gène peut être introduit tout simplement grâce à un plasmide ou au moyen d'un vecteur viral recombinant du même type que celui utilisé pour faire pénétrer à l'intérieur des cellules-cibles le gène codant pour l'élément marqueur. On utilise de préférence des vecteurs permettant à la fois l'expression de l'élément marqueur et la présentation de l'antigène d'intérêt en association avec les antigènes du CMH. Ainsi, les cellules-cibles peuvent être coinfectées par deux vecteurs, l'un pour l'expression de l'élément marqueur, l'autre pour l'expression de l'antigène particulier. On a remarqué que, pour permettre une bonne expression à la fois de l'élément marqueur et de l'antigène particulier, il était préférable de procéder simultanément à la co-infection des cellules-cibles par les deux vecteurs. Grâce à cette méthode, il est possible de sensibiliser les cellules-cibles par l'antigène entier et non plus seulement par des peptides épitopes. Il suffit, en effet, de pouvoir disposer du gène codant pour l'antigène entier et de l'introduire dans un vecteur approprié tel que les virus Pox modifiés mentionnés précédemment.

De façon particulièrement avantageuse selon l'invention, on utilise non pas deux vecteurs pour l'expression d'une part de l'élément marqueur et d'autre part de l'antigène particulier, mais un seul vecteur digénique permettant la co-expression des deux protéines d'intérêt. Par vecteur digénique, on entend un vecteur porteur de 2 gènes qui lui sont étrangers, chaque gène étant sous contrôle d'un promoteur propre ou, dans le cas particulier de vecteur bicistronique, les 2 gènes étant sous contrôle d'un seul promoteur. L'utilisation de vecteur digénique permet d'être absolument certain que toutes les cellules qui seront lysées grâce à une activité cytotoxique spécifique des cellules effectrices seront bien mises en évidence. L'utilisation d'un seul vecteur digénique est particulièrement intéressante dans le cas où il est nécessaire d'utiliser de faibles multiplicités d'infection (MOI).

Selon une autre caractéristique de l'invention, on améliore encore la sensibilité de la méthode en utilisant non pas un vecteur digénique mais un vecteur trigénique comprenant en outre un gène codant pour une molécule favorisant l'adhésion de la cellule effectrice à la cellule-cible. On peut ainsi introduire un gène codant par exemple pour la molécule ICAM-1 ou la molécule LFA-3, ce qui permet lorsque les cellules effectrices sont des cellules T cytotoxiques de renforcer leur potentiel de lyse des cellules-cibles.

Lors de la mise en oeuvre de la méthode selon la présente invention, les cultures de cellules sont réalisées de façon classique, avec les milieux appropriés définis selon la nature des cellules utilisées ; de même, le dosage de l'élément marqueur libéré dans le milieu extracellulaire après action des cellules effectrices sur les cellules-cibles est réalisé selon les techniques habituelles. Cependant, lorsque l'élément marqueur est la β-galactosidase, on a remarqué qu'on obtenait de particulièrement bons résultats en utilisant une méthode de dosage basée sur l'activité de cette enzyme avec le substrat luminescent AMPGD (Adamantyl methoxyphényl galactosidase 1,2 dioxétane).

On peut, pour déterminer le pouvoir cytotoxique des cellules effectrices, procéder de la manière suivante : on détermine d'une part la quantité d'élément marqueur libéré spontanément par un certain nombre de cellules-cibles auxquelles on n'a pas ajouté de cellules effectrices (libération spontanée) ; on détermine d'autre part la quantité d'élément marqueur libéré dans le milieu extracellulaire par un même nombre de cellules-cibles (sans cellules effectrices) qui sont totalement lysées, par exemple au moyen d'un ajout de solution IGEPAL et une incubation à l'étuve (libération totale) ; et on détermine en outre la quantité d'élément marqueur libéré dans le milieu extracellulaire par un même nombre de cellules-cibles mises en contact avec les cellules effectrices (libération effectrices).

Ces déterminations permettent alors d'obtenir des résultats qui vont conduire à l'expression d'un pourcentage de cytotoxicité, calculé de la façon suivante :

$$\text{Pourcentage de cytotoxicité} = \frac{\text{libération effectrices - libération spontanée}}{\text{libération totale - libération spontanée}} \times 100$$

Les résultats obtenus avec la méthode de dosage selon l'invention, bien que ne pouvant pas être considérés comme des valeurs quantitatives absolues, notamment à cause des étapes de stimulation in vitro des cellules effectrices, permettent cependant d'effectuer valablement des comparaisons entre les différentes cellules effectrices testées quant à leur pouvoir de cytotoxicité.

Exemple 1

Evaluation de la cytotoxicité spécifique dirigée contre la nucléoprotéine NP du virus de la grippe, de cellules spléniques de souris immunisées contre la nucléoprotéine NP, au moyen de cellules-cibles sensibilisées par des antigènes peptidiques.

a) préparation des cellules effectrices

On immunise des souris Balb/c femelles âgées de 8 à 10 semaines, en intramusculaire avec de l'ADN plasmidique contenant le gène codant pour la nucléoprotéine NP du virus de la grippe (souche 60/68) sous le contrôle du promoteur iel du CMV et du terminateur BGH (hormone de croissance bovine), à raison de 20 μg d'ADN par souris. Un rappel est effectué 2 à 3 semaines plus tard.

2 semaines après le rappel, on prélève les rates stérilement dans du milieu RPMI 1640 (fourni par Gibco sous la réf. 42401-018) et supplémenté par du SVF (Sérum de Veau Foetal) à 2 % et du GSPβ (Glutamine, Streptomycine, Pénicilline, β mercaptoéthanol). Les rates sont ensuite broyées puis passées sur un tamis dont le seuil de coupure est de 70 μm. Les cellules sont centrifugées et reprises par de la solution de Gey's (3 ml par rate) qui a la propriété d'hémolyser les globules rouges. Après 5 min de contact dans la glace, les cellules sont lavées 3 fois en milieu RPMI 10 % SVF GSPβ, et ajustées à $3.10^7$ cellules/ml de milieu.

On obtient des cellules stimulantes à partir de splénocytes de souris de même génotype non immunisées qui sont lavées dans du milieu RPMI contenant du GSPβ mais pas de SVF et dont le culot après centrifugation est incubé pendant 3 heures à 37°C sous 5 % de $CO_2$ avec le virus de la grippe NIB16, à 100 HAU (Hemagglutination units) pour $10^6$ cellules. Les cellules sont ensuite lavées trois fois dans du RPMI 10% SVF GSPβ puis ajustées à $2.10^7$ cellules stimulantes/ml. On met ensuite les cellules en culture dans un rapport de 2 cellules stimulantes pour 3 cellules effectrices, avec une concentration finale de $2,5.10^6$ cellules/ml. Les cellules sont incubées pendant 5 jours à 37°C sous $CO_2$ à 5%.

b) préparation des cellules-cibles

On utilise des cellules de la lignée P815 provenant d'un mastocytome de souris DBA/2. Ces cellules sont histo-compatibles ($H2\text{-}K^d$) avec les cellules de souris Balb/c. Les cellules sont ajustées à $10^7$ cellules/ml dans du RPMI 2 % SVF GSPβ puis infectées par le vecteur vP425 pendant 1 heure à 37°C avec une multiplicité d'infection de 20 PFU pour une cellule. Le vecteur vP425 provient du virus de la vaccine (WR) et contient le gène codant pour la βgalactosidase d'E.coli sous contrôle du promoteur late 11kDa de la vaccine. Il est obtenu selon la technique décrite dans l'article "Expression in Recombinant Vaccinia Virus of the Equine Herpesvirus 1 Gene Encoding Glycoprotein gp13 and Protection of Immunized Animals", Guo et al, Journal of Virology, Oct.1989, p. 4189 - 4198. Les cellules infectées sont ajustées à 2.10$^5$ cellules/ml dans du RPMI 10% SVF GSPβ.

Afin de permettre à ces cellules P815 de présenter à leur surface l'épitope H2-K$^d$ restreint de l'antigène NP en association avec les molécules de classe I du CMH, ces cellules sont incubées en même temps avec le peptide NP qui est un peptide de 11 acides aminés correspondant aux AA 147 à 158 de la nucléoprotéine du virus de la grippe Influenza A PR8 en solution à 2 g/l (fournie par Néosystème Laboratoire Strasbourg). Les cellules-cibles utilisées pour mettre en évidence la cytotoxicité non spécifique sont elles incubées avec le peptide HA qui est un peptide de 10 acides aminés correspondant aux AA 189 à 199 de la protéine HA2 du virus de la grippe Influenza A Singapoor en solution à 2g/l (fournie également par Néosystème Laboratoire Strasbourg).

Les peptides NP ou HA sont ajoutés aux cellules à raison de 20 μg de peptide pour 10$^6$ cellules. Après une nuit d'incubation à la fois en présence du vecteur vP425 et du peptide NP ou HA, les cellules sont lavées 3 fois dans du RPMI 10 % SVF GSPβ. Après centrifugation, on ajoute à nouveau sur le culot cellulaire 20 μg de peptide pour 10$^6$ cellules. Après une nouvelle incubation d' 1 heure 30, les cellules sont lavées avec du RPMI 10 % SVF GSPβ. Une partie de ces cellules est ajustée à la concentration de 5.10$^4$ cellules/ml en RPMI 10% SVF GSPβ. Une autre partie est mise en contact avec 300 μCi de $^{51}$Cr pour 10$^6$ cellules pendant 1 heure à l'étuve. Ces cellules sont dites doublement marquées. On prépare également des cellules P815, non infectées par le vecteur vP425 mais incubées avec l'un des peptides et marquées au $^{51}$Cr.

### c) mise en plaque

Les cellules-cibles et effectrices sont distribuées en microplaque 96 puits à fonds ronds FALCON 3077 dans des rapports de 100, 33, 11, 3 cellules effectrices pour une cellule-cible et incubées 4 h à 37°C sous 5% de $CO_2$. Les cellules effectrices sont distribuées dans un volume de 100 μl à raison de 5.10$^5$ cellules par puits en triplicat, puis diluées en cascade de raison trois. Les cellules-cibles sont distribuées à raison de 5.10$^3$ cellules par puits.

### d) dosage

Après les 4 heures d'incubation, la libération totale et la libération spontanée sont définies pour chaque type de cellules-cibles.

La libération spontanée correspond à la libération des cellules-cibles en absence de cellules effectrices. Dans ces puits, on détecte le chrome ainsi que la βgalactosidase présents dans le surnageant.

La libération totale est mesurée après une lyse totale des cellules-cibles (en absence de cellules effectrices), lyse réalisée par addition de 30 μl d'une solution d'IGEPAL 0,125 % et une incubation de 15 min à l'étuve.

Le dosage du chrome radioactif est réalisé en transférant 30 μl du surnageant des cellules dans des plaques de comptage Lumaplate 96 (PACKARD). Ces plaques contiennent un scintillant solide permettant la mise en évidence de l'émission d'électrons d'Auger ; ces électrons sont détectés par un compteur de rayonnements béta (TopCount-PACKARD). Les résultats obtenus sont exprimés en Coups Par Minute (CPM).

Le dosage de la βgalactosidase est réalisée par "Glow Luminescence" au moyen du kit Galacto-Light Plus™ (de TROPIX). On distribue 30 μl de surnageant des cellules dans des plaques MicroFLUOR™ 96 puits (de DYNATECH) ; on y ajoute 100 μl de substrat Galacton-Plus™, dilué au 1/100e dans du Galacto-Light™ Reaction Buffer Diluent. On incube les plaques 45 min à température ambiante, à l'abri de la lumière. On ajoute 100μl d' "Accelerator" et on lit la plaque au TopCount après un temps d'attente de 5 min. Les résultats sont exprimés en Coups Par Seconde (CPS).

Que le dosage soit un dosage du chrome radioactif libéré ou un dosage de la β galactosidase libérée, on exprime toujours le résultat en pourcentage de cytotoxicité calculée de la façon suivante :

$$100 \times \frac{\text{libération effectrices - libération spontanée}}{\text{libération totale - libération spontanée}}$$

Ce résultat est calculé pour chaque valeur (100, 33, 11 ou 3) du ratio cellules effectrices/cellules-cibles.

### e) résultats

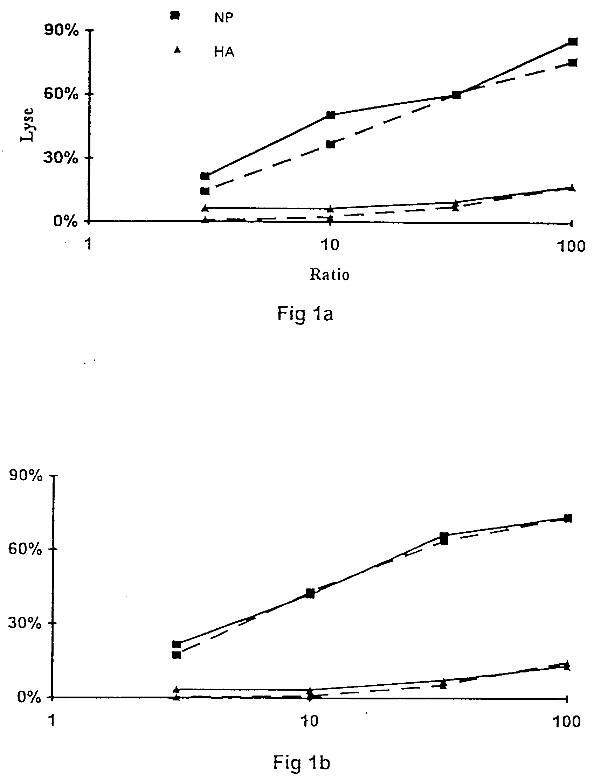

Les résultats obtenus sont illustrés aux figures 1a et 1b, sur lesquelles les traits en pointillés sont relatifs au dosage de la radioactivité et les traits pleins au dosage de la luminescence. Les résultats indiqués par un carré sont obtenus avec des cellules-cibles incubées avec le peptide NP alors que les résultats indiqués par un triangle sont obtenus avec des cellules-cibles incubées avec le peptide HA.

Les courbes de la figure 1a illustrent les résultats obtenus avec des cellules-cibles simplement marquées, soit par la βgalactosidase, soit par du chrome radioactif. Dans ce cas, on voit que les résultats obtenus sont similaires dans les 2 cas.

Les courbes de la figure 1b illustrent les résultats obtenus avec des cellules-cibles doublement marquées, à la fois par la βgalactosidase et par le chrome radioactif.

Ici encore, la quasi-superposition des courbes obtenues montre que les résultats obtenus avec la méthode selon l'invention peuvent être directement comparés avec ceux obtenus avec une méthode selon l'art antérieur.

D'autre part, les valeurs indiquées à la figure 1b étant à peu près identiques à celles de la figure la, on peut en conclure que le double marquage des cellules-cibles ne perturbe pas les résultats obtenus.

Exemple 2

Evaluation de la cytotoxicité spécifique dirigée contre la nucléoprotéine NP du virus de la grippe, de cellules spléniques de souris immunisées contre la nucléoprotéine NP, au moyen de cellules-cibles infectées par un vecteur codant pour la nucléoprotéine NP.

a) préparation des cellules-effectrices

Identique à celle de l'exemple 1.

b) préparation des cellules-cibles

On utilise, comme dans l'exemple 1, des cellules P815 que l'on ajuste à une concentration de $5.10^6$ cellules/ml en RPMI 2% SVF GSPβ et que l'on infecte soit avec le vecteur vP425 et le vecteur vP261, soit avec le vecteur vP425 et le vecteur vP 1214, avec une MOI (Multiplicity of Infection) de 10 pour chaque vecteur ;
Le vecteur vP425 est celui utilisé dans l'exemple 1;
Le vecteur vP261 provient également du virus de la vaccine et contient le gène codant pour la nucléoprotéine NP du virus de la grippe sous contrôle du promoteur I3L de la vaccine ; il est obtenu selon la technique décrite dans l'article "Characterization and immunological properties of influenza A virus nucleoprotein (NP) : cell-associated NP isolated from infected cells or viral NP expressed by vaccinia recombinant virus do not confer protection", Stitz and al, Journal of General Virology (1990);
Le vecteur vP1214 provient aussi du virus de la vaccine et contient le gène codant pour la protéine pp65 du Cytomegalovirus humain sous contrôle du promoteur H6 de la vaccine. Il est obtenu selon la méthode décrite dans la demande WO96/39491 ; ce vecteur permet l'expression de la pp65, protéine interne de la capside du CMV à la surface des cellules infectées dans le contexte du CMH ; les cellules infectées par ce vecteur permettent d'évaluer la cytotoxicité non spécifique dans ce test.

Après l'infection, les cellules sont incubées 1 heure à 37°C, puis elles sont ajustées à une concentration de $2.10^5$ cellules/ml dans du RPMI 10 % SVF GSPβ et incubées une nuit à l'étuve. Le lendemain, les cellules sont lavées 3 fois dans du RPMI 10 % SVF GSPβ, puis marquées au chrome radioactif de la même façon que cela a été décrit à l'exemple 1. Elles sont ensuite ajustées à une concentration de $5.10^4$ cellules/ml dans du RPMI 10 % SVF GSPβ.

c) mise en plaque

Identique à celle de l'exemple 1

d) dosage

La détermination de l'activité cytotoxique est faite à la fois par luminescence et par dosage de la radioactivité selon les techniques décrites à l'exemple 1.

e) résultats

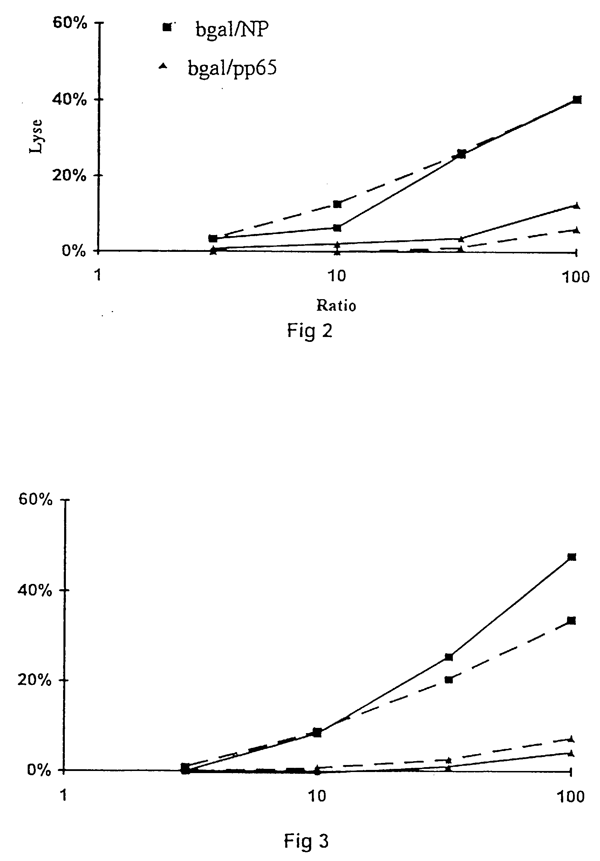

Les résultats obtenus sont illustrés sur la figure 2, sur laquelle les traits pleins sont relatifs au dosage de la luminescence et les traits pointillés au dosage de la radioactivité ; les carrés identifient des résultats obtenus avec des cellules-cibles infectées par un vecteur permettant d'exprimer les épitopes de la protéine NP, alors que les triangles identifient des résultats obtenus avec des cellules-cibles infectées par un vecteur permettant d'exprimer la protéine pp65 du CMV humain.

Ici encore, les résultats obtenus avec la méthode selon l'invention sont similaires à ceux obtenus avec une méthode selon l'art antérieur.

Exemple 3

Evaluation de la cytotoxicité spécifique dirigée contre la nucléoprotéine NP du virus de la grippe, de cellules spléniques de souris immunisées contre la nucléoprotéine NP, au moyen de cellules-cibles infectées par un double recombinant codant à la fois pour la β galactosidase et pour la nucléoprotéine NP.

a) préparation de cellules effectrices

Identique à celle de l'exemple 1.

b) préparation des cellules-cibles

On utilise comme dans l'exemple 1 des cellules P815 que l'on ajuste à une concentration de $5.10^6$ cellules/ml en RPMI 2% SVF GSPβ et que l'on infecte soit avec le vecteur vP1429, soit avec le vecteur vP1363B, avec une MOI de 10 ; le vecteur vP1429 est un vecteur digénique qui provient du virus de la vaccine (WR) et contient le gène codant pour la βgalactosidase d'E.coli sous contrôle du promoteur H6 et le gène codant pour la nucléoprotéine NP du virus de la grippe sous contrôle du promoteur I3L de la vaccine ;

Le vecteur vP1363B provient également du virus de la vaccine (WR) et contient le gène codant pour la βgalactosidase d'E.coli et le gène codant pour la protéine pp65 du Cytomégalovirus humain chacun sous contrôle d'un promoteur H6 de la vaccine. Les cellules infectées par ce vecteur permettent de déterminer la cytotoxicité non spécifique.

Après l'infection, les cellules sont incubées 1 heure à 37°C, puis elles sont ajustées à une concentration de $2.10^5$ cellules/ml dans du RPMI 10 % SVF GSPβ et incubées une nuit à l'étuve. Le lendemain, les cellules sont lavées 3 fois dans du RPMI 10 % SVF GSPβ, puis marquées au chrome radioactif de la même façon que cela a été décrit à l'exemple 1. Elles sont ensuite ajustées à une concentration de $5.10^4$ cellules/ml dans du RPMI 10 % SVF GSPβ.

c) mise en plaque

Identique à celle de l'exemple 1

d) dosage

La détermination de l'activité cytotoxique est effectuée à la fois par luminescence et par dosage de la radioactivité selon les techniques décrites à l'exemple 1.

e) résultats

Les résultats obtenus sont illustrés à la figure 3, où comme à la figure 2, les traits pleins sont relatifs au dosage de la luminescence et les traits pointillés au dosage de la radioactivité. Les carrés identifient des résultats obtenus avec des cellules-cibles infectées par le double-recombinant permettant l'expression de la βgalactosidase et des épitopes de la nucléoprotéine NP alors que les triangles identifient les résultats obtenus avec des cellules-cibles infectées par le vecteur permettant l'expression de la βgalactosidase et des épitopes de la protéine pp65 du CMV humain.

Les courbes obtenues montrent que la méthode selon l'invention permet d'obtenir des résultats à peu près identiques à ceux obtenus grâce à la méthode de l'art antérieur.

En outre, si on compare ces résultats obtenus en utilisant des cellules-cibles infectées par un vecteur digénique permettant la co-expression par la cellule-cible à la fois de l'élément marqueur et de l'antigène vis-à-vis duquel on veut évaluer l'activité cytotoxique spécifique des cellules effectrices avec les résultats obtenus en utilisant des cellules-cibles doublement infectées, d'une part par un vecteur permettant l'expression de l'élément marqueur et d'autre part par un vecteur permettant l'expression de l'antigène vis-à-vis duquel on veut évaluer l'activité cytotoxique spécifique (figure 2), on remarque qu'aux multiplicités d'infection utilisées, ils sont du même ordre, ce qui signifie notamment que, d'une part l'expression d'un gène n'interfère pas avec l'expression de l'autre gène lorsqu'on utilise un vecteur digénique et, d'autre part que lorsqu'on fait une double infection, les cellules sont bien infectées simultanément par les 2 vecteurs.

Exemple 4

Evaluation de la cytotoxicité spécifique dirigée contre la protéine pp65 du Cytomegalovirus (CMV) humain, de lymphocytes humains provenant d'un donneur CMV+, au moyen de cellules-cibles infectées par un vecteur codant pour la protéine pp65 du CMV.

a) <u>préparation des cellules effectrices</u>

On récupère un échantillon de sang total d'un donneur connu pour être CMV+ qui a été infecté naturellement par le Cytomegalovirus.

Les lymphocytes (PBL) sont séparés par centrifugation sur gradient de densité (Ficoll-Eurobio) dans des tubes "Barrier Centrifuge Tube" (Sigma Réf. B-6156) à 800 g pendant 5 min. Après avoir aspiré le plasma, on récupère l'anneau de mononucléaires qu'on lave par 2 fois dans du liquide physiologique, puis on effectue un dernier lavage en milieu RPMI 1640 (fourni par GIBCO sous la Réf. 42401-018). Les cellules sont, ensuite, comptées et diluées à la concentration de $10^7$ cellules/ml.

On prépare des cellules stimulantes à partir de lymphocytes issus de sang périphérique du même donneur qui sont infectés par un vecteur provenant du Canarypox virus et contenant le gène codant pour la protéine pp65 du Cytomegalovirus humain (le vCP260 fourni par VIROGENETICS à une concentration de $1,5.10^9$ PFU/ml) ; l'infection est réalisée à une MOI (Multiplicity of Infection) de 5. Après incubation pendant 1 h 30, les cellules sont lavées en milieu CML supplémenté par 10 % de Sérum Veau Foetal (le milieu CML est constitué du milieu RPMI fourni par GIBCO sous la Ref 42401-018 supplémenté en sodium pyruvate 100 mM, en AA non essentiels et en Hepes 10 mM).

Les cellules effectrices sont mises en culture avec les cellules stimulantes dans un rapport de 1 cellule stimulante pour 4 cellules effectrices, en présence d'IL-7 à 330 U/ml. La concentration des cellules dans la culture au départ est de $1,5.10^6$ cellules/ml.

Les cellules sont incubées pendant 5 jours à 37°C sous $CO_2$ à 5 %..

7 jours après la mise en culture, une partie des cellules effectrices est restimulée par addition d'IL.2 (Interleukine 2 Recombinante fournie par GENZYME) à raison de 10 U/ml. Les cellules sont, dans ce cas, à nouveau incubées pendant 5 jours à 37°C sous $CO_2$ à 5 %.

b) <u>préparation des cellules-cibles</u>

On prépare une lignée de cellules EBV à partir des cellules PBL lavées en milieu RPMI sans sérum et ajustées à une concentration de $2.10^6$ cellules/ml.

Après centrifugation, on élimine le milieu de culture. On reprend le culot par 400 µl de RMPI sans sérum et on ajoute 400 µl de surnageant EBV.

On incube les cellules 1 h 30 à 37°C sous $CO_2$ à 5 % puis on ajoute du milieu RPMI supplémenté en SVF à 2 % de façon à avoir une suspension de $10^6$ cellules/ml.

On ajoute ensuite de la cyclosporine à une concentration finale de 1 µg/ml.

On ajuste la concentration des cellules à $2.10^6$ cellules/ml en milieu CML supplémenté en SVF que l'on infecte soit avec le vecteurvP425 et le vecteur vP1214, soit avec le vecteur vP425 et le vecteur vP1238, avec une MOI de 10 pour chaque vecteur ; le vecteur vP425 est celui utilisé dans l'exemplel, le vecteur vP1214 est celui utilisé dans l'exemple2, mais dans cet exemple, les cellules infectées par ce vecteur permettent d'évaluer la cytotoxicité spécifique ; Le vecteur vP1238 provient du virus de la vaccine et contient le gène codant pour la protéine pp150 du Cytomegalovirus humain sous contrôle du promoteur H6 de la vaccine ; les cellules infectées par ce vecteur permettent, dans cet exemple, d'évaluer la cytotoxicité non spécifique.

L'infection des cellules est réalisée par une incubation à 37°C pendant 1 h 30. Ensuite, les cellules sont ajustées à $10^6$ cellules/ml dans du milieu CML contenant 10 % de SVF et incubées une nuit à l'étuve.

Le lendemain, les cellules sont lavées 3 fois dans du milieu CML contenant 10 % de SVF et sont ramenées à une concentration de $5.10^5$ cellules/ml.

Une partie des cellules-cibles est, ensuite, marquée au $^{51}Cr$, à raison de 100 µCi pour $10^6$ cellules, afin d'obtenir comme à l'exemple 1, des cellules doublement marquées.

Les cellules marquées sont ajustées directement à une concentration de $5.10^4$ cellules/ml en CML 10 % SVF GSP.

c) <u>mise en plaque</u>

Identique à celle de l'exemple 1.

d) <u>dosage</u>

La détermination de l'activité cytotoxique est faite à la fois par luminescence et par dosage de la radioactivité selon les techniques décrites à l'exemple 1.

e) <u>résultats</u>

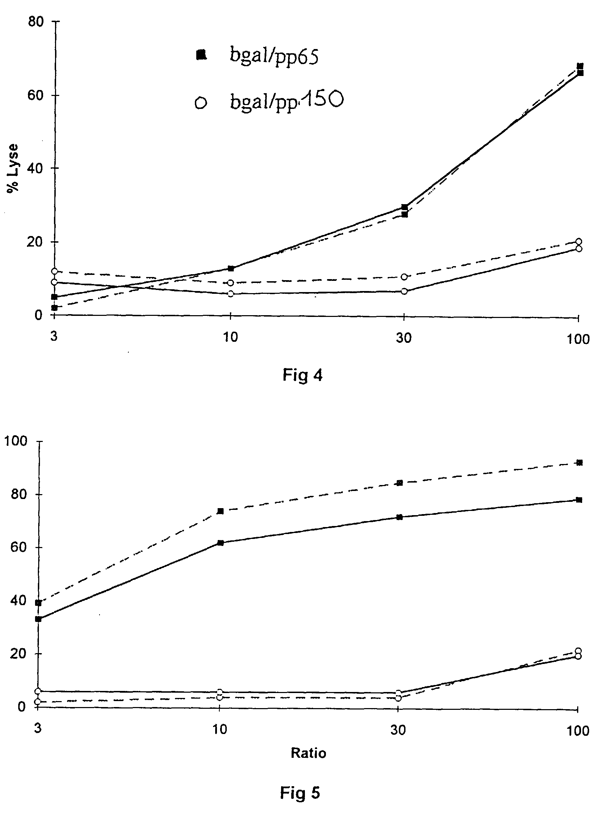

Les résultats obtenus avec les cellules effectrices n'ayant été stimulées qu'une fois sont présentés à la figure 4 et les résultats obtenus avec les cellules effectrices ayant été stimulées 2 fois sont présentés à la figure 5. Sur ces figures, les traits pleins sont relatifs au dosage de la luminescence et les traits pointillés au dosage de la radioactivité.

Les carrés identifient des résultats obtenus avec des cellules-cibles infectées par un vecteur permettant d'exprimer la protéine pp65 du CMV, alors que les ronds identifient des résultats obtenus avec des cellules-cibles infectées par un vecteur permettant d'exprimer la protéine pp150 du CMV.

Ici encore, les résultats obtenus avec la méthode selon l'invention sont similaires à ceux obtenus avec une méthode selon l'art antérieur.

**Revendications**

1. Méthode de mise en évidence de l'activité cytotoxique de cellules effectrices, selon laquelle on met en contact les cellules effectrices et des cellules-cibles et on apprécie la cytotoxicité par dosage d'un élément marqueur présent dans le cytoplasme des cellules-cibles et libéré dans le milieu extracellulaire en cas d'activité cytotoxique des cellules effectrices vis-à-vis des cellules-cibles , caractérisée en ce qu'on introduit au préalable dans les cellules-cibles au moins un gène codant pour ledit élément marqueur.

2. Méthode selon la revendication 1, caractérisée en ce qu'on introduit en outre dans les cellules-cibles au moins un gène codant pour un antigène particulier vis-à-vis duquel on souhaite évaluer l'activité cytotoxique des cellules effectrices.

3. Méthode selon la revendication 2, caractérisée en ce que le gène codant pour ledit élément marqueur et le gène codant pour ledit antigène particulier sont introduits dans les cellules-cibles au moyen d'un vecteur digénique.

4. Méthode selon une des revendications précédentes, caractérisée en ce qu'elle consiste en outre à introduire dans les cellules-cibles au moins un gène codant pour une molécule favorisant l'adhésion des cellules effectrices aux cellules-cibles.

5. Méthode selon la revendication précédente, caractérisée en ce que les gènes sont introduits dans les cellules-cibles au moyen d'un vecteur trigénique.

6. Méthode selon une des revendication précédentes, caractérisée en ce que ledit élément marqueur est une enzyme.

7. Méthode selon la revendication précédente, caractérisée en ce que l'enzyme est la β-Galactosidase.

8. Méthode selon une des revendications précédentes, caractérisée en ce que les cellules cibles sont des cellules humaines.

9. Méthode selon une des revendications précédentes, caractérisée en ce que le vecteur de transmission du gène codant pour ledit élément marqueur est un vecteur viral dérivé de la vaccine.

Fig 1a

Fig 1b

Fig 2

Fig 3

EP 0 844 483 A1

Fig 4

Fig 5

**RAPPORT DE RECHERCHE EUROPEENNE**

Numéro de la demande

EP 97 42 0214

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | US 4 314 026 A (B. DESCAMPS-LATSCHA.)<br>* le document en entier *<br>--- | 1-9 | G01N33/50<br>C12Q1/02 |
| A | US 5 180 662 A (M. V. SITKOVSKY.)<br>* le document en entier *<br>--- | 1-9 | |
| A | EP 0 605 370 A (CELLIFE BIOTECNOLOGIE PER LA VITA)<br>* le document en entier *<br>--- | 1-9 | |
| A | WO 92 14839 A (IMPERIAL CANCER RESEARCH TECHNOLOGY LIMITED)<br>* le document en entier *<br>----- | 1-9 | |

| | DOMAINES TECHNIQUES RECHERCHES (Int.Cl.6) |
|---|---|
| | G01N<br>C12Q |

Le présent rapport a été établi pour toutes les revendications

| Lieu de la recherche | Date d'achèvement de la recherche | Examinateur |
|---|---|---|
| LA HAYE | 23 février 1998 | Griffith, G |